# EUROPEAN PATENT APPLICATION

(11) **EP 3 788 956 A1**
(43) Date of publication of application: **10.03.2021**
(21) Application number: 19195135.9
(22) Date of filing: 03.09.2019
(51) Int. Cl.: A61B 5/08, G16H 50/20, A61C 17/22

(54) **CONTROLLER**

(71) Applicant: Koninklijke Philips N.V., 5656 AG Eindhoven (NL)
(72) Inventor: GERHARDT, Lutz Christian, 5656 AE Eindhoven (NL); MENA BENITO, Maria Estrella, 5656 AE Eindhoven (NL); VISWESWARA, Ashoka Sathanur, 5656 AE Eindhoven (NL); RONDA, Cornelis Reinder, 5656 AE Eindhoven (NL); SCHEFFERS, Lucas Petrus Henricus, 5656 AE Eindhoven (NL)
(74) Representative: de Haan, Poul Erik

(57) **Abstract**

There is provided a controller for a fluid -detection system, wherein the controller is configured to: determine if a user is interacting with the fluid detection system, wherein the determining comprises at least one of: determining whether a user is within a predetermined distance of the fluid detection system; determining whether a user is in physical contact with the fluid detection system; determining whether a fluid sensor of the fluid detection system is in a mouth of a user; and control the temperature of the fluid sensor of the fluid detection system based on the result of the determination. There is also provided a fluid based detection system comprising the controller, a device comprising the controller, a remote device comprising the controller, and a computer implemented method for controlling a fluid detection system, the method comprising the steps of: determining if a user is interacting with the fluid detection system, wherein the determining comprises at least one of: determining whether a user is within a predetermined distance of the system; determining whether a user is in physical contact with the fluid detection system; determining whether a fluid sensor of the fluid detection system is in a mouth of a user; and controlling the temperature of the fluid sensor of the fluid detection system based on the result of the determination.

## Description

### FIELD OF INVENTION

This invention relates to a controller and particularly but not exclusively relates to a controller for a fluid-detection system.

### BACKGROUND OF THE INVENTION

Halitosis, also known as "bad breath" or "oral malodour," is identified in up to 50% of the adult population. The malodour originates from proteins being broken down into volatile sulphur compounds (VSCs) or diamines (putrescine, cadaverine).

Besides its social effect, pathological halitosis is considered one of the most common symptoms/indications of oral pathologies. In up to 85-95% of cases, the malodour originates from the oral cavity itself and/or an otorhinolaryngological source. However, in the remaining 5-15% of cases, the source is non-oral and the malodour can indicate a distal systemic disease, and is therefore termed extra-oral pathological halitosis. The cause could be, for example, an acute or chronic kidney, liver, gastrointestinal or respiratory disease, diabetes, or cancer.

To date there are several tools available to detect and quantify specific compounds related to halitosis. For example, sulphur monitors are commercially available which show an acceptable correlation with organoleptic scores determined from smelling the breath at approximately 10 cm from the mouth of a person. However, many monitors are not suitable to be integrated into hand-held devices due to the challenges of miniaturization, are not able to discriminate between different VSCs or diamines (low specificity), and/or have low sensitivity.

In addition to the above commercially available devices, fluid detection systems based on chemoresitivity such as semiconducting metal oxide (SMOX) gas sensor technologies are also being developed to detect bad breath compounds in halitosis patients. A disadvantage of these sensors is that they require high sensor surface temperatures (150-400 °C) which results in high energy consumption, an effect which is particularly undesirable for portable or handheld devices which are not connected to a continuous energy supply. Furthermore, if the sensor is exposed to decomposing surrounding gases while at the high temperatures, precipitates form on the reactive sensor surface, severely reducing the operational lifetime of SMOX sensors. The precipitates may also compromise the accuracy of readings taken by a sensor, and a reduction in accuracy may not be apparent to the user, thus resulting in a user being provided with, and utilizing, incorrect information.

### SUMMARY OF THE INVENTION

It would be advantageous to provide a system which addresses some of the issues outlined above. To better address particular sensor lifetime/degradation and power management concerns, there is provided a controller for a fluid -detection system. All of the aspects below may provide the advantage of reduction in power consumption and/or reduction in degradation of a fluid sensor.

According to an embodiment of an aspect there is provided a controller for a fluid -detection system (for example, a SMOX based detection system, particle detection system), wherein the controller is configured to: determine if a user is interacting with the fluid detection system, wherein the determining comprises at least one of: determining whether a user is within a predetermined distance of the fluid detection system; determining whether a user is in physical contact with the fluid detection system; determining whether a fluid sensor (where fluid is e.g. gas or liquid) of the fluid detection system is in a mouth of a user; and control the (operating) temperature of the fluid sensor of the fluid detection system based on the result of the determination.

The fluid sensor may be a gas sensor, and/or may be a chemoresistive sensor. The determining if a user is interacting with the fluid detection system may be based on a predetermined trigger condition or device usage which comprises at least one of: a time trigger condition, device usage information of a device comprising the fluid sensor (for example, drivetrain current), use steps associated with actual device usage (for example, following a two minute brushing cycle of a toothbrush), user behaviour patterns within the predetermined distance of the system (e.g. a distance of less than 1cm, between 1 and 10 centimeters, between 10 and 100cm, a distance greater than 1m, the distance may be a distance set by a user or manufacturer), user interaction patterns between the user and a device of the fluid detection system (for example, interaction of a user with a toothbrush comprising the fluid sensor), user interaction patterns between the user and a device including the fluid sensor (for example, picking up the device), user interaction with a remote device (for example, interaction of a user with a smart phone), an AI algorithm based trigger configured to predict whether a user is intending to use the fluid sensor (for example, based on patterns of behavior of a user). For example, the determining may include determining whether a user is behaving in a manner which indicates that the user is about to interact with the fluid-detection system. For example, daily routines of the user, such as entering the bathroom in the morning, may be detected in order to determine that the user is about to interact with the fluid-detection system. The operating temperature of the sensor may be a temperature in the range of 100-500°C, for example.

The device usage information may comprise information relating to at least one of: brushing time, brushing motor current, airflow passing over the device or sensor, change in surface temperature of the fluid sensor or device, impedance of the fluid sensor or a device including the fluid sensor, capacitance of the fluid sensor or a device including the fluid sensor, humidity adjacent to the device, pressure on the device, force applied to the device, proximity to the device, or light intensity to which the device (or the device sensor) is exposed (for example, using a photodiode that measures darkness-lightness differences (if device is put in mouth the light intensity decreases)). The fluid sensor described herein may be any type of fluid sensor that is capable of sensing gas or liquid, and that requires its temperature to be elevated to operate.

The controller may be configured to activate heating of the fluid sensor when it is determined that a user is interacting with the fluid detection system.

The controller may be configured to deactivate heating of the fluid sensor when at least one of the following conditions is fulfilled: when no interaction of the user with the fluid detection system is detected, a predetermined time after activation of the fluid sensor (for example, 10 seconds, 1 minute, etc.) a trigger condition that indicates that further interaction of the user with the fluid detection system is unlikely (for example, the trigger condition may be the switching off of ambient light when leaving a bath room, a geo fence indication that a user has left the house etc.).

The controller may be configured to select a mode of operation of the fluid sensor based on the interaction of the user with the fluid detection system, and wherein the controller may be configured to control the temperature of the fluid sensor based on the mode of operation. For example, the way in which the user is interacting with the fluid detection system may indicate whether the user is intending to use the fluid sensor (e.g. removal of a device comprising the fluid sensor from a charging station may indicate the intent of the user to use to fluid sensor. In this case, heating of the fluid sensor may therefore be activated in anticipation of a user being about to use the fluid sensor and, once it is determined that the fluid sensor is in the mouth of the user, detecting or measurement may be performed).

The mode of operation of the fluid sensor may include at least one of: a sensing mode, a sensor surface regeneration mode (e.g. a cleaning mode), an on mode, and an off mode. For example, the sensing mode may be a mode in which data acquisition is started (once the fluid sensor is in the mouth of the user and has been heated to its operating temperature). The sensor surface regeneration mode may be a mode in which the fluid sensor surface is heated over a prolonged time (e.g. 1-5 min) and or at higher temperature (e.g. 10-20 % higher) than the operating temperature to regenerate the sensing surface of the fluid sensor. This may involve heating the sensor surface to a temperature slightly higher than the operating temperature. The on mode may be the activation of the fluid detection system, and may involve heating of the fluid sensor (or the surface of the fluid sensor) to operating temperature. The off mode may be the deactivation of the fluid detection system, and may involve stopping heating of the fluid sensor (or the surface of the fluid sensor).

The cleaning mode may be selected when it is determined that the user is interacting with the fluid detection system (through physical contact of the user with the fluid detection system, when the system decides that the user is not using the device within a predetermined amount of time, e.g. based on AI interpretation of user behavior, based on daily routines of the user) and the fluid sensor is not in the mouth of the user; and the sensing mode may be selected when it is determined that the user is interacting with the fluid detection system and the fluid sensor is in the mouth of the user (according to intended use of the device).

According to a further aspect, there is provided a fluid based detection system comprising the controller, further comprising the fluid sensor.

The fluid detection system may further comprise at least one of: a device-user interaction sensor configured to monitor device-user interactions, a device usage sensor configured to monitor contextual information during device usage, a remote device comprising the controller, a device comprising the controller. For example, the motion of the device comprising the fluid sensor may be used to determine when the user has picked up the device, and when the user has put the fluid sensor in their mouth. Alternatively or additionally, the device usage sensor may, for example, use information about the use of the device, such as drivetrain current, to determine how long the device has been used for, to determine when detecting should commence. The controller may be comprised in the same device as the fluid sensor. The controller may be provided in a server, and control the operation of the fluid sensor wirelessly using any known wireless technology. The controller may be provided in a mobile phone.

According to a further aspect there is provided a device comprising the controller.
The device may comprise one of: an oral cleaning device, a toothbrush, an oral irrigator, a flossing device, an interdental cleaning device, a halitosis detection device, a mouthpiece, a handle or body of an oral cleaning device, a handle or body of a toothbrush, a handle or body of an oral irrigator, a handle or body of a flossing device, a handle or body of an interdental cleaning device, a handle or body of a halitosis detection device, a handle or body of a mouthpiece, a remote device, or any combination thereof. The controller may be provided in the same device as the fluid sensor. The handle or body may be configured to receive a head of the device.

According to a further aspect there is provided a remote device comprising the controller. The remote device may be a smart phone, a smart watch, a smart mirror, a server, a tablet, a computer, a house/home, and/or vehicle.

According to a further aspect there is provided a computer implemented method for controlling a fluid detection system, the method comprising the steps of: determining if a user is interacting with the fluid detection system, wherein the determining comprises at least one of: determining whether a user is within a predetermined distance of the system; determining whether a user is in physical contact with the fluid detection system; determining whether a fluid sensor of the fluid detection system is in a mouth of a user; and controlling the temperature of the fluid sensor of the fluid detection system based on the result of the determination.

There may be provided a computer program comprising instructions which, when the program is executed by a computer, cause the computer to carry out the computer implemented method outlined above.

### BRIEF DESCRIPTION OF THE DRAWINGS

Embodiments of the present disclosure may take form in various components and arrangements of components, and in various steps and arrangements of steps. Accordingly, the drawings are for purposes of illustrating the various embodiments and are not to be construed as limiting the embodiments. In the drawing Figures, like reference numerals refer to like elements. In addition, it is to be noted that the Figures may not be drawn to scale.
Fig. 1 illustrates a controller and a fluid sensor for a fluid detection system according to an example;
Fig. 2 illustrates an oral care device in which the fluid detection system is implemented according to an example;
Fig. 3 illustrates the steps implemented by the controller according to an example;
Fig. 4 illustrates an always-on configuration applied to the fluid detection system according to an example;
Fig. 5 illustrates the fluid detection system according to an example;
Fig. 6 illustrates a flow chart of the operation of the fluid sensor according to an example;
Fig. 7 illustrates a flow chart of the operation of the fluid sensor according to another example;
Fig. 8 illustrates a flow chart of the operation of the fluid sensor according to a further example;
Fig. 9 illustrates the detection of user interaction of the fluid sensor according to an example;
Fig. 10 illustrates the detection of user interaction of the fluid sensor according to an example; and
Fig. 11 illustrates a user using a device comprising the fluid sensor according to an example.

### DETAILED DESCRIPTION OF EMBODIMENTS

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein may be used in the practice or testing of the embodiments.

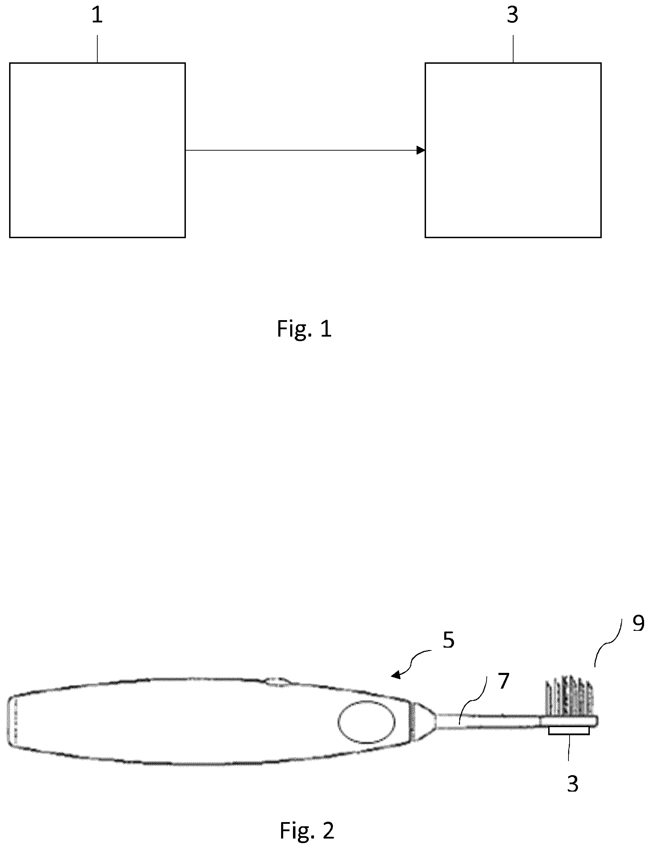

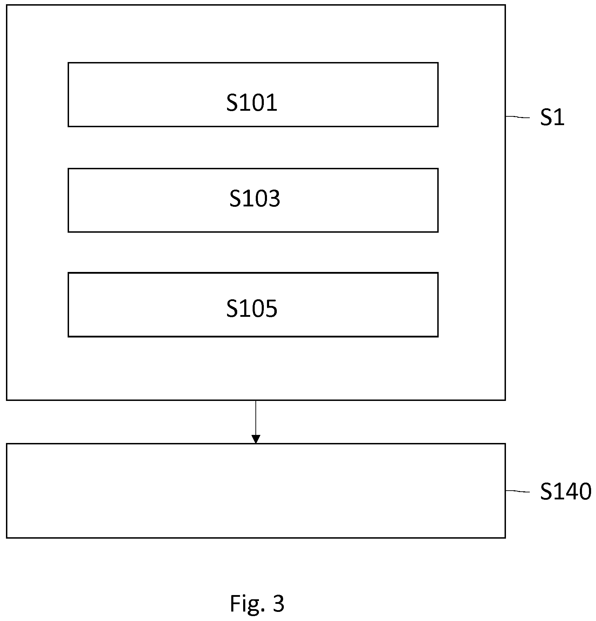

Fig. 1 illustrates a controller 1 for a fluid detection system. The controller 1 is configured to determine if a user is interacting with the fluid detection system and control the temperature of a fluid sensor 3 of the fluid detection system based on the result of the determination. Fig. 2 illustrates a device which may comprise the fluid sensor 3. The fluid sensor may be a SMOX sensor. In this example, the device is a power toothbrush, however, it should be appreciated that devices to which the system may be applied include connected oral healthcare devices such as Bluetooth enabled power toothbrushes; toothbrushes connected via WLAN, 4G, 5G, coded light; irrigator-toothbrush combinations; interdental cleaning devices, oral irrigators, flossing devices, halitosis detection devices and/or mouthpieces, nebulisers or other handheld devices into which a user blows. The system may also be implemented in any other personal care device such as facial cleaners, shavers, or hair trimmers in close vicinity to the mouth or nose nostril where breath sensing can be performed. Furthermore, the system may be implemented in any other handheld device in which fluid sensors (such as SMOX sensors) are provided. In this example, the fluid sensor 3 is provided on a head 7 of the toothbrush 5, and is arranged on a side of the head opposite the bristles 9 (backside of platen). The controller may be provided in the device. However, the controller may alternatively be provided in a remote device, such as a mobile device (mobile phone), in a server or the like. The controller may signal to the fluid sensor, or a processor that controls the fluid sensor, and the temperature of the fluid sensor may be controlled on the basis of the signaling. The steps taken by the controller are illustrated by the flow chart shown in Fig. 3.

Fig. 3 shows a flow chart of steps taken by the controller. At step S1, the controller determines if the user is interacting with the fluid detection system. The determination includes at least one of: determining whether a user is within a predetermined distance of the fluid detection system (S101); determining whether the user is in physical contact with the fluid detection system (S103); and determining whether a fluid sensor of the fluid detection system is in a mouth of a user (S105). The determining may include determining whether a user is behaving in a manner which indicates that the user is about to interact with the fluid-detection system. For example, daily routines of the user, such as entering the bathroom in the morning, may be detected in order to determine that the user is about to interact with the fluid-detection system. Determining whether the user is within a predetermined distance of the fluid detection system may involve determining whether the user is in a room which comprises the fluid sensor (for example, in a bathroom). The location of a user (relative to the device) measured, for example, using (centimetre-accurate) GPS (global positioning system), IPS (indoor positioning system) may be used to determine whether the user is within a predetermined distance of the system. Other proximity sensing technologies may be also used such as measurement of capacitance changes. The controller then controls the temperature of the fluid sensor of the fluid detection system based on the result of the determination (S140). For example, the fluid sensor may be heated, or cooled (e.g. by stopping heating), based on the result of the determination.

Heating a fluid sensor consumes energy. As an example, current SMOX sensors require about 2-250 mW average power to heat the sensor to perform 1 measurement over 2 seconds. However, even if low power consumption sensors are used in a handheld device, continuous operation of a fluid sensor will lead to a significant power consumption. Particularly in the case of a SMOX sensor, continuous operation may also lead to degradation of the fluid sensor surface. To detect a gas (such as a target gas), a SMOX sensor may determine a change of the sensor resistance caused by a target gas reacting with oxygen adsorbed at the surface of the metal oxide, (oxygen is adsorbed onto the surface of the SMOX sensor). However, when the fluid sensor is at a higher temperature, precipitates may form on the reactive sensor surface, e.g. due to gases that decompose at the surface of the sensor and do not react away completely. The more precipitates that form on the sensor surface, the fewer the reactions of the sensor surfaces with the target gas, and therefore the less change in sensor resistance, occurs, and the sensitivity of the sensor is reduced. In addition, the presence of precipitate may also result in sensor drift. Therefore, by determining if a user is interacting with the fluid detection system, and controlling the temperature of the chemically reactive fluid sensor (surface) on the basis of the determination, power can be conserved, and additionally degradation of the fluid sensor may be slowed or reduced.

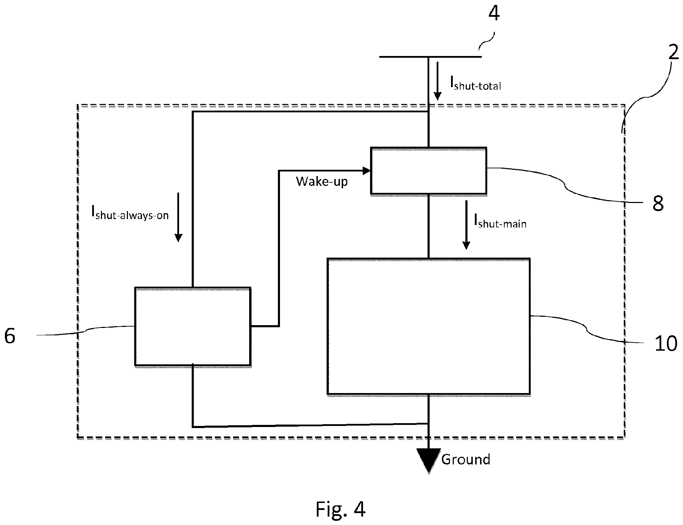

For example, the determination may be based on a predetermined trigger condition or device usage. For instance, in a power management circuit for low power electronics (Fig. 4), the sensor may be activated at certain time intervals or using an external trigger. In such cases, the fluid-detection system may spend most of its time in shut-down mode to conserve its energy and prevent the reactive sensor surface from degradation. Alternatively or additionally, an "always-on" block 6 which is continuously switched-on to react to a periodic or an asynchronous external trigger to wake-up the main electronics (which is otherwise in low power shut-down mode) to perform its function may be used. The energy consumption of an always-on block may be very small compared to the main electronics in active mode, which may help in minimizing energy expenditure of the main electronics.

Fig. 4 illustrates an example of a power management system which is configured to reduce power consumption of an electronic system 2 by controlling current flow to high power consumption elements of the system using an always-on block 6. The power management system may be comprised in an oral care device, such as one comprising a fluid sensor, and may be configured to reduce power consumption of the fluid sensor. The electronic system 2 comprises a main electronics block to control the sensor electronics 10 which is connected to a power supply 4, via a power switch block 8, and ground. An always-on block 6 is connected in parallel to the main electronics block 10, and is connected between the power supply 4 and ground. The always-on block 6 is also connected to the power switch block 8.

In this example, the power dominant main electronic block 10 is powered via power switch block 8. The power switch block 8 is switched-off when the electronic system 2 goes into shut-down (or off) mode. By switching off the power switch block 8, the total shut down current Iₛₕᵤₜ₋ₜₒₜₐₗ contributed by the main electronic block 10 Iₛₕᵤₜ₋ₘₐᵢₙ is significantly reduced. The shut-down current contribution of the always-on block 6 _{Ishut-always-on} becomes a significant part of the total shut-down current Iₛₕᵤₜ₋ₜₒₜₐₗ of the electronic system 2 when the electronic system goes into shut-down mode. The always-on block 6 is configured to receive a wake up trigger or activation signal which indicates that the main electronics block 10 should be activated. When the always-on block 6 receives a wake-up signal, the always-on block 6 causes power switch block 8 to be switched on, so that current is provided to the main electronics block 10.

Thus, the total current required during sleep mode may be substantially reduced as less current is supplied to the main electronics block, and the current required to operate the main electronics block 10 may only be supplied when a wake-up signal has been received by the always-on block 6. Thus, total battery time of a device, for example an oral care device comprising the fluid sensor, operating using the aforementioned power management system may be improved. In an example, the system may be used to only supply power (e.g. heat up) a fluid sensor when a wake-up signal is received which has a positive effect on sensor lifetime.

The fluid detection system may comprise at least two verification steps. For example, the fluid sensor may be activated when a device user interaction or user distance detection occurs, as is described above. However, the fluid detection system may contain an additional verification step (device usage detection), which may include an automated mode selection (for example, the fluid sensor entering either a sensing/measurement mode or a cleaning mode). This verification step may be used to detect unintentional or false activation (switching on) of the system. The selection of the mode of operation of the fluid sensor may be triggered in response to one or more of the following series of events and/or triggers including pre-determined conditions such as time-triggers (e.g. end-of brushing cycle such as 2 minutes or end of a tongue cleaning cycle), audible triggers (such as recognizing drivetrain noise, bathroom noise such as the user spitting or rinsing the mouth) usage behavior or usage recognition triggers (personalized sensors which monitor actual brushing time e.g. through motor current measurement and change thereof, detection of airflow passing over the fluid sensor, a sudden change in surface temperature, impedance, relative humidity and/or pressure/force on the device mouthpiece or a part of the device introduced in oral cavity), AI based events/triggers, for example algorithms which predict when measurement is to be taken (e.g. based on device orientation/location sensing over time with respect to an initial stationary position (e.g. in charging station)), smart phone software controlled events (e.g. change of angle, velocity on first look on the smart phone or connected bathroom mirror using gesture detection).

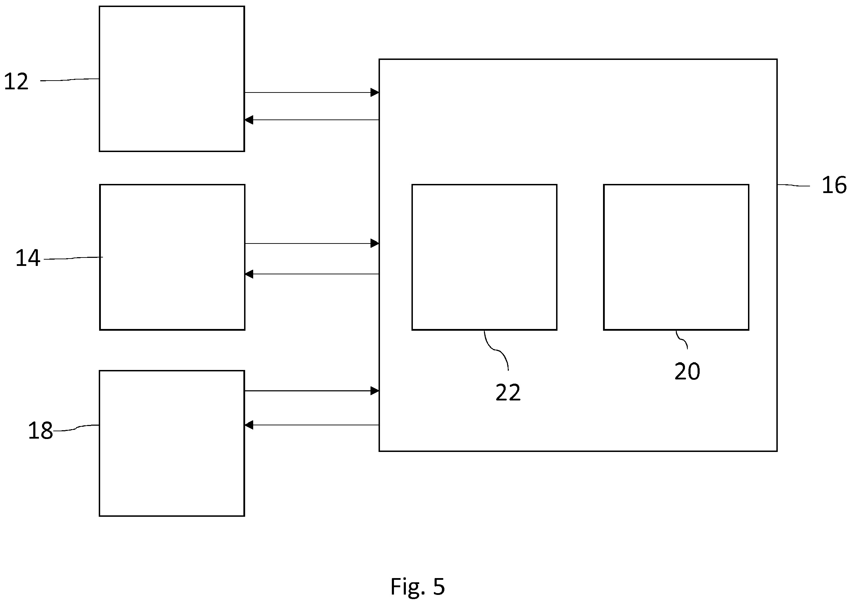

Fig. 5 illustrates a SMOX-based detection system according to an example. Fig. 5 shows a device-user interaction sensor 12 and a device usage sensor 14 connected (directly or indirectly) to a controller 16. A fluid sensor 18 such as a SMOX sensor (or another sensor which typically operates at high temperatures and undergoes sensor degradation at these high temperatures) is also connected to the controller 16. The controller 16 is configured to determine if a user is interacting with the fluid sensor 18 based on signaling from the device-user interaction sensor 12 and the device usage sensor 14.

The device-user interaction sensor 12 (which may be an active sensor or a passive sensor) is configured to generate one or more signals to control a mode of operation of the fluid detection system. An active sensor is a sensing device that requires an external source of power to operate; active sensors contrast with passive sensors, which simply detect and respond to some type of input from the physical environment (e.g. piezo, self-powered triboelectric sensor). The device-user interaction sensor 12 generates a signal in response to a determination that a user is interacting with the fluid detection system, for example, based on the generation or activation of events or trigger(s), e.g. mechanical triggers, a time trigger, event. The device-user interaction sensor may comprise a communication unit (which may comprise an antenna or the like) to transmit a signal to the controller 16 and communicate with external devices. For example, the device-user interaction sensor may transmit a start (switch on) or end (switch off) signal for starting or ending heating and/or sensing (data acquisition) by the fluid sensor 14 to the controller 16.

The device usage sensor 14 may be configured to provide contextual information on the actual device usage (for example, if the fluid sensor is in a mouth of a user) to be able to detect whether the device is intentionally being used by a user. For example, the device-user interaction sensor 12 may detect that the user is in the same room as the fluid sensor, and the device usage sensor 14 may detect that a device comprising the fluid sensor is in the hand, or mouth of a user, indicating that the device is being intentionally used by a user. Conversely, if the device-user interaction sensor 12 detects that the user is in the same room as the fluid sensor but the device is not in the hand and/or mouth of a user, then it is indicative that the user does not intend to use the fluid sensor.

The controller 16 may comprise a microcontroller processor 20. The microcontroller processor 20 may be configured to form logic gate circuits that use Boolean algebra to perform mathematical operations allowing switching between different operation modes of the fluid sensor. For example, the fluid sensor may switch between a sensing mode, a sensor surface regeneration mode, an on mode or an off (sleep) mode. The controller 16 may further comprise a switch 22 configured to cause the fluid sensor 14 to switch between different operation modes.

Triggers which indicate if a user is interacting with the fluid detection system (for example with a device of the system and/or the fluid sensor) may be threshold based (e.g. brushing force exceeds a certain threshold value or is deviating from zero for few seconds). Alternatively or additionally, a conditional process (if-then-else) can be used to switch on or off the fluid detection system or change modes. Triggers can be, for example, time based, completion of sensing, when nothing has happened during some time (such as 5 mins), when sensor signal or drive train motor current are constant within specified limits, and when sensor activation and sensing triggers described are not present anymore (for example, time differentiation or variation of device user interaction and device usage sensor signals over time may be used to switch off the system).

In the sensing mode described herein, measurement of VSC / data acquisition and data communication may be performed by the fluid detection system. The cleaning mode described herein may involve heating the fluid sensor to a certain temperature above the optimum operating temperature to desorb molecules from sensor surface. A mode auto-selection between a sensing mode and a cleaning mode may be initiated/triggered automatically, for example, if the fluid detection system is activated unintentionally or falsely, the cleaning mode can be selected rather than the sensing mode. A sensor signal input on the actual device usage (e.g. capacitive or temperature sensor in handle, or change of drivetrain current over short time of 1 minute) or other contextual information may be used to describe logic states which may be used to switch between the sensing mode and the cleaning mode (select different modes). Triggers and conditions to enter the cleaning mode can be time based, for example, after a predetermined period of sensing time, when it is determined that a user is not interacting with the system (for example, when no interaction has occurred happened during a predetermined time period) and/or when the fluid sensor signal or motor current of the device are measured to be constant within specified limits.

The system could be configured to perform a cleaning step before a measurement is taken, for example directly after the wake-up to reset the sensor (baseline calibration), if the device has not been used for long period of time or in varying conditions e.g. after travel to a tropical region. The necessity to clean the fluid sensor, and therefore selecting or not selecting the cleaning mode, can be calculated by the time the sensor has been on, the magnitude of the last signal from the fluid sensor (an atypical sensor current signal may indicate that cleaning is required), life time of the fluid sensor, average temperature etc. The fluid detection system may be switched off (or go into low power mode) after either sensing or cleaning has been performed.

A data log may record the number of (heating) cycles, interval cycles such as time-temperature (heating) intervals of the fluid sensor. The information may then be used to estimate the possible deterioration of the system, for example to determine if the sensor needs to be replaced. This could also be used to improve the accuracy of the measurements (so that false positives of bad breath are not given).

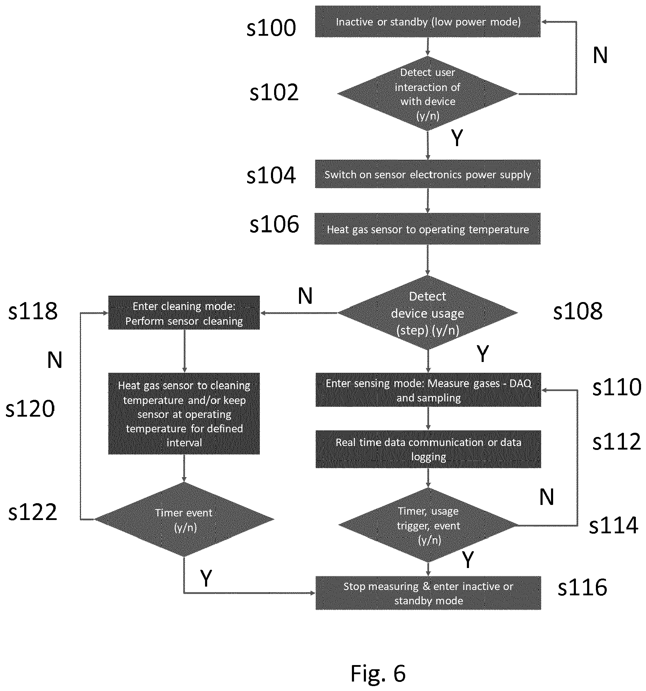

Fig. 6 shows a flow chart for controlling the fluid detection system illustrated by Fig. 5, in particular an automated slowly responding fluid detection system. For example, Fig. 6 shows the information flow of a multimodal fluid detection system and method with automated mode selection (e.g. SMOX fluid detection system) for breath analysis in a handheld device. The flow chart shown in Fig. 6 may be particularly appropriate for slow-responding (slowly heating > 1 minute) automated fluid detection systems with separate sensor activation and sensing verification steps.

At step S100, the fluid sensor is in an inactive or standby (low power) mode. At S102, the device-user interaction sensor 12 is configured to detect whether a user is interacting with a device comprising the fluid sensor. If it is determined that the user is not interacting (N) with the device, the fluid sensor is maintained in an inactive or standby mode. If user interaction with the device is detected (Y), the process proceeds to step S104. At this point, the fluid sensor electronics power supply is switched on, and at step S106, the fluid sensor is heated to an operating temperature. At step S108, the device usage sensor 14 is then used to determine whether the device comprising the fluid sensor is being used in a manner that would indicate that the user wishes to perform fluid detection/measurement (for example, it is determined whether the fluid sensor is in the mouth of a user). Where it is determined that the device comprising the fluid sensor is being used in a manner that indicates the intent of the user to detect or measure fluid (Y), the fluid sensor enters a sensing mode, where fluid may be measured (DAQ and sampling) (step S110). At step S112, the data gathered by the fluid sensor in the sensing mode is stored and/or is transmitted. Data is collected until a signal is given for the data collection to stop (S114). The signal may indicate that a predetermined amount of time has passed since the data collection or the start of the sensing, a usage trigger such as movement of the fluid sensor out of the mouth of a user, or an event (e.g. after completion of brushing, a pre-defined lag time if a false alarm occurred, or where no brushing was carried out as e.g. baby needed attention or an unforeseen phone call occurred). Until the signal is given (Y) steps S110 and S112 are repeated. When a signal is given for the data collection to stop as a result of the timing, usage trigger or event (Y), the fluid sensor stops measuring/collecting data and enters an inactive or standby mode (S116).

At step S108, if device usage, such as the fluid sensor being in the mouth of the user, is not detected (N), the device instead enters a cleaning mode (sensor surface regeneration mode) to perform cleaning of the sensor (S118). At step S120, the fluid sensor is heated to a cleaning temperature (such as 440 °C, above a typical operating temperature) or the fluid sensor is held at a typical operating temperature (such as 300 - 400 °C). The sensor may be held at the cleaning temperature for a period of time, such as 1min-5mins). At step S122, it is determined whether a predetermined amount of time has elapsed since sensor cleaning has started. If the predetermined amount of time has not elapsed (N), the process moves back to step S118, and steps S118 to S122 are repeated. If the predetermined amount of time has elapsed (Y), the process moves to step S116, and the fluid sensor enters an inactive or standby mode.

As is shown in Fig. 6, the sensor electronics is automatically switched on and the sensor operating temperature is regulated/controlled once it has been determined that a user is interacting with the device. In a subsequent verification step, based on the actual device usage such as whether the device is in the mouth of a user, the sensor operation mode is auto regulated, entering either a sensing/measurement mode or a cleaning/regeneration mode before the fluid detection system goes back to the inactive/sleep mode.

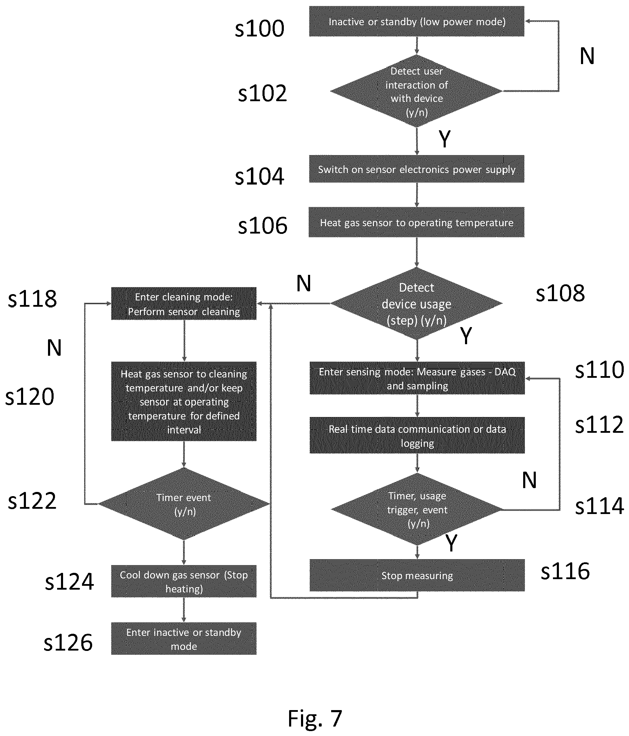

Fig. 7 shows the flow chart as illustrated in Fig. 6, with additional steps. In Fig. 7, after S116 in which measurement by the fluid sensor ends, the fluid sensor then implements the function of step S118, in which the fluid sensor enters a cleaning mode and performs sensor cleaning after the in mouth measurement. The process then moves through steps S120 and S122 as described above in relation to Fig. 6. Once the predetermined amount of time determined in S122 has elapsed (Y), the process moves to step S124, in which the fluid sensor is cooled down (for example, the fluid sensor stops heating). The fluid sensor then enters an inactive or standby mode (S126).

Thus, the sensor electronics is automatically switched on and the sensor operating temperature is regulated/controlled based on user interaction with the device. In a subsequent verification step based on user interaction indicating actual device usage the sensor operation mode is auto regulated, entering either a sensing/measurement mode followed by a cleaning mode, or a cleaning/regeneration mode, before the fluid detection system goes back to the inactive/sleep mode.

The controller may therefore be configured to operate the fluid sensor using either of these process flows, and may select between them.

In the examples of Figs. 6 and 7, the sensor is automatically activated through a direct or remote device-user interaction while the sensor operation mode is automatically selected based on the actual device usage detection. If proper device usage is detected (for example, where the sensor is in the mouth of a user), the system enters the sensing mode. If not, it performs a cleaning action by further heating the sensor to the cleaning temperature or keeping it at the operating temperature for a predetermined time period.

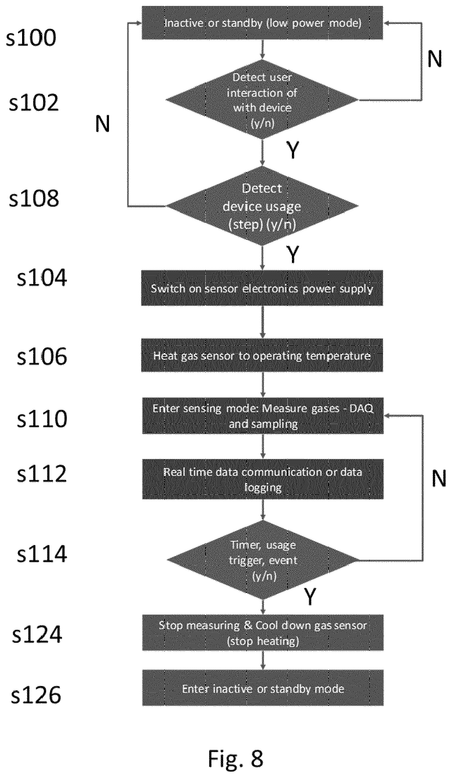

Fig. 8 shows a flow chart for control of the fluid detection system illustrated by Fig. 5, in particular an automated fast responding fluid detection system. Fast-responding fluid detection systems may be capable of heating to operating temperature in under one second or hundreds of milliseconds.

In the process illustrated by Fig. 8, steps S100 and S102 are the same as in the process shown in Fig. 6. For example, at step S100, the fluid sensor is in an inactive or standby (low power mode). At S102, the device-user interaction sensor 12 is configured to detect whether a user is interacting with a device comprising the fluid sensor. If it is determined that the user is not interacting with the device (N), the fluid sensor is maintained in an inactive or standby mode. If user interaction with the device is detected (Y), the process in this case instead proceeds to step S108. At step S108, the device usage sensor 14 is then used to determine whether the device comprising the fluid sensor is being used to detect fluid (for example, it is determined whether the fluid sensor is in the mouth of a user). Where it is determined that the device comprising the fluid sensor is being used to detect fluid (Y), the process proceeds to step S104. At this point, the sensor electronics power supply is switched on, and at step S106, the fluid sensor is heated to an operating temperature. Once the operating temperature is reached, the fluid sensor enters a sensing mode, where fluid may be measured (DAQ and sampling) (step S110). At step S112, the data gathered by the fluid sensor in the sensing mode is stored and/or is transmitted. Data is collected until a signal is given for the data collection to stop (S114). The signal may be in the form of a timer which indicates that a predetermined amount of time has passed since the data collection or sensing started, a usage trigger such as movement of the fluid sensor out of the mouth of a user, or an event such as completion of brushing or a pre-defined lag time if no brushing was carried out. Until the signal is given (N) steps S110 and S112 are repeated. When a signal is given for the data collection to stop as a result of the timer, usage trigger or event, the fluid sensor stops measuring/collecting data and the fluid sensor cools (stops heating) S124. The fluid sensor then enters an inactive or standby mode S126.

As is outlined in the process of Fig. 8, for fast-responding fluid detection systems, the fluid detection system may be automatically activated and the fluid sensor surface automatically heated to operating temperature and measurements taken if two conditionals are fulfilled - user interaction with the device and device usage detection. If proper device usage is detected (fluid sensor inside the mouth of the user), the system may directly enter the sensing mode. It is noted that the process is not limited to the process shown in Fig. 8, and the process may also include the steps of performing a cleaning action, for example, the steps relating to the cleaning mode shown in Figs. 6 or 7.

The processes outlined above, which switch a sensor from a stand-by mode to an active (heating) mode (or cleaning mode) and switch it off after use, may provide the advantage that the fluid sensor is only heated when needed, resulting in both increased sensor lifetime and reduced power consumption (in particular, if energy hungry handheld devices are used and cannot be continuously connected to the charging station).

As mentioned above, it may be determined that a user is interacting with the fluid detection system and the temperature of the fluid sensor may be controlled based on the result of the determination. It may be determined that a user is interacting with the fluid detection system based on a predetermined trigger condition or device usage information (for example, by using information on how the device is being used by the user). Such trigger conditions/device usage may include any of a time trigger condition (such as the end of a two minute time period for brushing of the teeth, where the sensor is heated, and the measurement is taken, once the brushing cycle has ended), device usage information of a device comprising the fluid sensor (for example, the motion of a toothbrush comprising the fluid sensor may indicate waiting for a measurement of the fluid sensor, such as a particular motion or angle of the toothbrush), use steps associated with actual device usage, user behavior patterns within the predetermined distance of the system, user interaction patterns between the user and a device of the fluid detection system, user interaction patterns between the user and a device including the fluid sensor, user interaction with a remote device, an AI algorithm based trigger configured to predict whether a user is intending to use the fluid sensor.

As an example of time triggered sensor activation, the fluid detection system may be activated (switched on) by a pre-determined timer setting, for example, inside a device comprising the fluid sensor. The timer may be programmed by the user either directly using the device or using a smart phone as a user interface, and a software-controlled trigger may be configured to wake up the device comprising the fluid sensor at pre-determined time points, e.g. 6 am and 9 pm. Depending on the further input of a device usage sensor, the fluid sensor may enter either a sensing or cleaning mode.

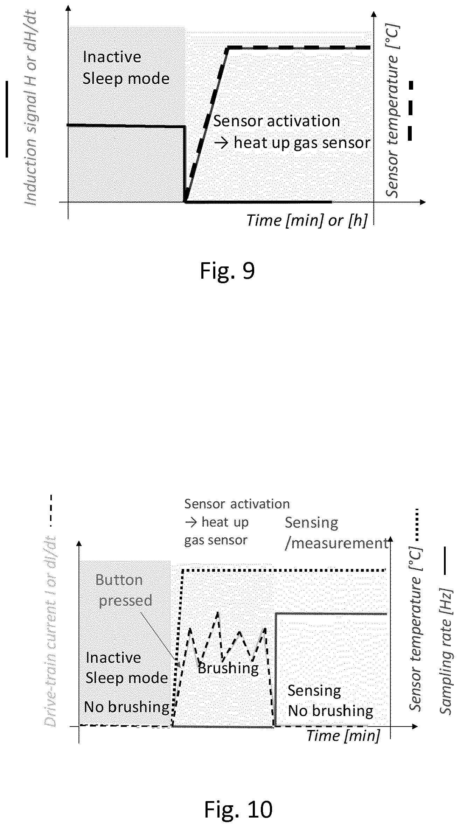

As an example of device-interaction pattern triggered and event triggered sensor activation, the fluid detection system may be activated by an event trigger. For example, when the user removes a device comprising the fluid sensor from a charging station of the device, a loss of the inductive charging signal can be used as a trigger to indicate that a user is interacting with the fluid detection system, and consequently the fluid detection system can be activated. An example of such a trigger is illustrated by the graph shown in Fig. 9. Fig. 9 shows an example of an indication signal pattern or signal change over time (time differentiation) as an indicator to activate the fluid detection system, and resultant sensor surface temperature signature. For example, as is shown in this graph, an induction signal being present (unbroken line) causes the fluid sensor to remain in an inactive mode. However, when the induction signal drops to zero (by removal of the device from the charging station), the sensor is activated (dashed line) and heats up to its operating temperature.

In another example, the fluid detection system may be activated by using optical (light) energy. The handheld device comprising the fluid sensor may also comprise a photo-sensitive element (e.g. photo-voltaic cell, photo sensor, photo-electric relay, photodiode). A photosensitive element may be, for example, integrated in the bottom of the toothbrush handle or at any other part of the handheld device. When the device is removed from a charging station or is moved so that the bottom of the device is exposed, light may be incident on the photo-sensitive element. A signal may be generated by the photo-sensitive element in response to the light, which may act as a trigger to indicate that a user is interacting with the device comprising the fluid sensor.

Coded light, or light with a unique wavelength per device, may be used to prevent false sensor activations if there are multiple fluid detection systems present. Furthermore, directional light may be applied, i.e. light may be shone only on the intended handheld device. This may be achieved using a camera installed in the bathroom (e.g. smart bathroom inside a mirror or in a room with vital signs camera) which is usable to determine the location of the device. This may also aid in directing the light beam to the right spot on the device (based on body parts recognition/identification: face, arm, leg), and may prevent activating other connected devices unintentionally. Unobtrusiveness for the user may be obtained by using near-infrared (NIR) light (i.e. light which is not visible for the human eyes).

Detection of a user entering the bathroom or movement of the device (e.g. by measuring transient sporadic changes in motion using accelerometers in the device or capacitance changes of the device, proximity sensors, or using a camera in a smart mirror) may also be used to activate the fluid sensor. Additionally or alternatively, a Bluetooth enabled smart device may be used to activate the sensing system using a standard Bluetooth protocol. For example, pairing of the device comprising the fluid sensor with a smart device when the devices are in proximity to one another may result in activation of the sensing system.

Artificial intelligence (AI) based events/triggers may also be used to activate fluid detection system. For example, algorithms may be used to predict when a fluid detection system is to be activated. The algorithms may determine whether the user is about to use the fluid detection system based on daily routines of the user, personalized device interaction (for example, the specific way a user uses a device) and usage patterns (the time of day/way in which a user uses the device during the week or at the weekend). Furthermore, the event/trigger may be related to the interaction of a remote device, such as a smart phone, with the fluid detection system. Activation of the fluid detection system can be established upon which the fluid detection system is automatically activated if a smart phone enabled connected oral care device is used and/or input from other smart home devices obtained.

Algorithms may be used to predict when measurements are to be taken, e.g. based on use of information from different activities (switching on the light in the bath room, visiting the toilet, locking the door, etc.). Smart home devices may also be used as providers of information which is usable to predict when a measurement is to be taken. For example, a self-learning thermostat may indicate the times that a user is likely to be home, the time that a user usually wakes up etc. Smart lighting may be usable to indicate at what time a user typically switches off lighting, whereby the switching off of the lighting (wake up light) or radio indicates the daily sleep/wake cycles of a user. An oral care device may sense when a user has attached, for example, a tongue brush to the oral care device, and may keep track of use frequency.

Data acquisition from the fluid sensor may also be triggered/started if pre-determined conditions or use steps associated with the actual device usage are fulfilled. For example, pressing a button on the device to start a brushing cycle may activate heating of the fluid sensor. A time-trigger indicating the end of the 2-minute brushing cycle or at the end of a tongue cleaning cycle may then be used as trigger events to begin data acquisition using the fluid sensor. These steps are illustrated in Fig. 10, which shows a graph of the processes involved.

In Fig. 10, device usage step and time triggered fluid sensing of a fluid sensor in an oral care device is illustrated. When no activity or interaction of the user is detected, the fluid detection system is in sleep mode. When a user beings to interact with the fluid detection system (for example, by brushing their tongue with the device), the fluid sensor is heated to a sensing temperature. While the user performs a two minute tongue brushing cycle, no sensing is performed. When the two minute brushing cycle has ended, e.g. at the end of the normal tongue brushing routine (2 mins elapsed), the fluid sensor is active (at a measurement temperature) and breath measurement is started.

Breath measurement after tongue brushing may be used to assess how successful the brushing has been. Furthermore, such a measurement may allow a prediction to be made of how long fresh breath may be maintained based on a VSC score (e.g. if the score is below 50 ppb, and the user is about to sleep which results in less saliva flow, the user may have 12 hours of fresh breath).

Data acquisition from the fluid sensor may also be started through device usage behavior or usage recognition triggers, e.g. detection of airflow (threshold flow rate/volume) passing over sensor, sudden/transient change in surface temperature, impedance, capacitance, relative humidity and/or pressure/force on a brush head, device mouthpiece or any part configured to be introduced into an oral cavity. For example, the determination of interaction of a user with the device, or triggering of the measurement of the fluid sensor, may be mouth temperature/humidity or airflow trigged (e.g. when a user breathes into a mouthpiece, the change of air flow or the force generated by pressing the lips (or tongue/ teeth) against the device is used as a trigger to start the data acquisition of the fluid sensor or activate the fluid detection system if a fast-responding sensor is used).

Algorithms may be used to trigger fluid sensing, taking measurements, and/or activating the fluid detection system. For instance, algorithms may be used to predict when a measurement can be started, for example, based on device orientation/location sensing over time with respect to an initial stationary position (e.g. device in charging station), or drivetrain current (to predict total brushing time).

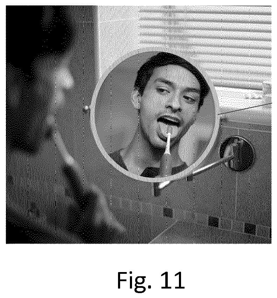

Smart (home) device based software controlled/detected events (e.g. change of angle of the device, eye lash blinking speed, velocity on first look on the smart phone or connected bathroom mirror using gesture detection, image analysis or even voice detection or voice command) may also be used as triggers to automatically start the fluid measurement. For example, a smart mirror may be used to detect when the device comprising the fluid sensor is on the tongue or in the mouth cavity of a user using image analysis methods, and using this information, software of a connected device can trigger the measurement (for example, see Fig. 11 which illustrates a smart mirror detecting a device on the tongue of a user).

The fluid sensor may be activated (i.e., the sensor surface may be heated) due to (remote) device-user interaction detection, such as in response to one or more of the following series of events and/or triggers including pre-determined conditions such as time-triggers (e.g. 6 am and 9 pm) set by a user, device-interaction patterns (e.g. touching power toothbrush, removing it from charging station, entering bath room, pressing a switch in bathroom), smart phone software controlled events (e.g. change of angle, velocity on first look on the smart phone), AI based events/triggers (e.g. algorithms predict when fluid detection system is to be activated, for example based on daily routines).

By controlling the temperature of the fluid sensor as described herein, the robustness to misuse may be enhanced (so that it is substantially error-proof, or provides fail-safe operation). Furthermore, automated regulation of switching between modes may provide easier use of the device comprising the fluid sensor for the user, and the fluid detection system can undergo an automatic cleaning step before and/or after measurement, which does not rely on the user and the fluid sensor will therefore be more consistently cleaned. Additionally, a reduction of data acquisition avoids wasting space in a memory, in addition to preventing power wastage (for example, where measurement / data acquisition (DAQ) is only started if device usage is recognized). There may also be no need for a mechatronic button on the device which may add manufacturing costs and is known to be a frequent source of failure due to contacting fatigue or corrosion issues as well source of hygiene issues (for example, mould growth in button interstices).

Although only a few exemplary embodiments have been described in detail above, those skilled in the art will readily appreciate that many modifications are possible in the exemplary embodiments without materially departing from the novel teachings and advantages of the embodiments of the present disclosure. The above-described embodiments of the present invention may advantageously be used independently of any other of the embodiments or in any feasible combination with one or more others of the embodiments

Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements. In a device or apparatus claim enumerating several means, several of these means may be embodied by one and the same item of hardware. The mere fact that certain measures are recited in mutually different dependent claims does not indicate that a combination of these measures cannot be used to an advantage.

In any of the above aspects, the various features may be implemented in hardware, or as software modules running on one or more processors. Features of one aspect may be applied to any of the other aspects.

The invention also provides a computer program or a computer program product for carrying out any of the methods described herein, and a computer readable medium having stored thereon a program for carrying out any of the methods described herein. A computer program embodying the invention may be stored on a computer-readable medium, or it could, for example, be in the form of a signal such as a downloadable data signal provided from an Internet website, or it could be in any other form.

## Claims

1. A controller for a fluid -detection system, wherein the controller is configured to:
determine if a user is interacting with the fluid detection system, wherein the determining comprises at least one of:
determining whether a user is within a predetermined distance of the fluid detection system;
determining whether a user is in physical contact with the fluid detection system;
determining whether a fluid sensor of the fluid detection system is in a mouth of a user; and
control the temperature of the fluid sensor of the fluid detection system based on the result of the determination.

2. The controller as claimed in claim 1, wherein the determining if a user is interacting with the fluid detection system is based on a predetermined trigger condition or device usage which comprises at least one of: a time trigger condition, device usage information of a device comprising the fluid sensor, use steps associated with actual device usage, user behavior patterns within the predetermined distance of the system, user interaction patterns between the user and a device of the fluid detection system, user interaction patterns between the user and a device including the fluid sensor, user interaction with a remote device, an AI algorithm based trigger configured to predict whether a user is intending to use the fluid sensor.

3. The controller as claimed in claim 2, wherein the device usage information comprises information relating to at least one of: brushing time, brushing motor current, airflow passing over the device or sensor, change in surface temperature of the fluid sensor or device, impedance of the fluid sensor or a device including the fluid sensor, capacitance of the fluid sensor or a device including the fluid sensor, humidity adjacent to the device, pressure on the device, force applied to the device, proximity to the device, or light intensity to which the device is exposed.

4. The controller as claimed in any preceding claim, wherein the controller is configured to activate heating of the fluid sensor when it is determined that a user is interacting with the fluid detection system.

5. The controller as claimed in any preceding claim, wherein the controller is configured to deactivate heating of the fluid sensor when at least one of the following conditions is fulfilled: when no interaction of the user with the fluid detection system is detected, a predetermined time after activation of the fluid sensor, a trigger condition that indicates that further interaction of the user with the fluid detection system is unlikely.

6. The controller as claimed in any preceding claim, wherein the controller is configured to select a mode of operation of the fluid sensor based on the interaction of the user with the fluid detection system, and wherein the controller is configured to control the temperature of the fluid sensor based on the mode of operation.

7. The controller as claimed in claim 6, wherein the mode of operation of the fluid sensor includes at least one of: a sensing mode, a sensor surface regeneration mode, an on mode, an off mode.

8. The controller as claimed in claim 7, wherein
the cleaning mode is selected when it is determined that the user is interacting with the fluid detection system and the fluid sensor is not in the mouth of the user; and
the sensing mode is selected when it is determined that the user is interacting with the fluid detection system and the fluid sensor is in the mouth of the user.

9. A fluid based detection system comprising the controller as claimed in any preceding claim, further comprising the fluid sensor.

10. The fluid detection system as claimed in claim 9, further comprising at least one of: a device-user interaction sensor configured to monitor device-user interactions, a device usage sensor configured to monitor device usage, a remote device comprising the controller, a device comprising the controller.

11. A device comprising the controller as claimed in any of claims 1 to 8.

12. The device as claimed in claim 11, wherein the device comprises one of: an oral cleaning device, a toothbrush, an oral irrigator, a flossing device, an interdental cleaning device, a halitosis detection device, a mouthpiece, a handle or body of an oral cleaning device, a handle or body of a toothbrush, a handle or body of an oral irrigator, a handle or body of a flossing device, a handle or body of an interdental cleaning device, a handle or body of a halitosis detection device, a handle or body of a mouthpiece, a remote device, or any combination thereof.

13. A remote device comprising the controller as claimed in any of claims 1 to 8.

14. A computer implemented method for controlling a fluid detection system, the method comprising the steps of:
determining if a user is interacting with the fluid detection system, wherein the determining comprises at least one of:
determining whether a user is within a predetermined distance of the system;
determining whether a user is in physical contact with the fluid detection system;
determining whether a fluid sensor of the fluid detection system is in a mouth of a user; and
controlling the temperature of the fluid sensor of the fluid detection system based on the result of the determination.

15. A computer program comprising instructions which, when the program is executed by a computer, cause the fluid detection system to carry out the method as claimed in claim 14.
